(19) **Europäisches Patentamt / European Patent Office / Office européen des brevets**

(11) **EP 4 524 987 A1**

(12) **EUROPEAN PATENT APPLICATION**
published in accordance with Art. 153(4) EPC

(43) Date of publication:
19.03.2025 Bulletin 2025/12

(21) Application number: 23855014.9

(22) Date of filing: 21.06.2023

(51) International Patent Classification (IPC):
*G16H 30/40* (2018.01)        *G16H 30/20* (2018.01)
*G16H 50/70* (2018.01)        *G16H 50/50* (2018.01)
*G06V 10/70* (2022.01)        *A61B 5/055* (2006.01)

(52) Cooperative Patent Classification (CPC):
A61B 5/055; G06V 10/70; G16H 30/20;
G16H 30/40; G16H 50/50; G16H 50/70

(86) International application number:
PCT/KR2023/008603

(87) International publication number:
WO 2024/039043 (22.02.2024 Gazette 2024/08)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 18.08.2022 KR 20220103608

(71) Applicant: Airs Medical Inc.
Seoul 06142 (KR)

(72) Inventor: JEONG, Keunwoo
Seoul 08788 (KR)

(74) Representative: Isarpatent
Patent- und Rechtsanwälte
Barth Hassa Peckmann & Partner mbB
Friedrichstraße 31
80801 München (DE)

(54) **LEARNING DATA GENERATION METHOD, COMPUTER PROGRAM AND DEVICE**

(57)    A training data generation method that is performed by a computing device including at least one processor according to an embodiment of the present disclosure includes: analyzing the signal strength and noise characteristics of one or more input images; determining whether an image pair having corresponding signal strength and noise characteristics is present among the one or more input images; and generating a training image and a label image according to the results of the determination based on a preset noise reduction target.

[FIG. 2]

EP 4 524 987 A1

**Description**

**Technical Field**

The present disclosure relates to a training data generation method, computer program and device, and more particularly to a method, computer program and device for generating training data using medical images.

**Background Art**

In general, a medical imaging apparatus is an apparatus that acquires a patient's body information and provides images. Such medical imaging apparatuses include X-ray imaging apparatuses, ultrasound diagnostic apparatuses, computed tomography apparatuses, and magnetic resonance imaging (MRI) apparatuses.

A magnetic resonance imaging (MRI) apparatus requires a considerable amount of time for imaging. Therefore, in the medical industry, acceleration imaging techniques to shorten MRI acquisition times are of significant importance. For accelerated MRI images to be usable in medical settings, they must include all necessary information about the subject while minimizing noise that could affect interpretation. Recently, due to this need, techniques have been developed to restore low-quality accelerated MRI images to high-quality states using artificial intelligence (AI).

To restore accelerated MRI images to high quality using AI, it is essential to effectively train the AI model. This requires not only high-quality input data but also corresponding high-quality label data. However, even if a certain level of accelerated MRI input data is obtained, there is a practical issue in that corresponding high-quality restored images are almost nonexistent. Therefore, securing the necessary training data is fundamental for building an AI model.

**Disclosure**

**Technical Problem**

The present disclosure is intended to overcome the problems of the prior art described above, and is directed to a training data generation method, computer program and device that generate training and label images according to the characteristics of input images and a noise reduction target.

However, objects to be achieved by the embodiments are not limited to the above-described object, and other objects may be present.

**Technical Solution**

According to an embodiment of the present disclosure for achieving the above-described object, there is disclosed a training data generation method that is performed by a computing device. The training data generation method includes: analyzing the signal strength and noise characteristics of one or more input images; determining whether an image pair having corresponding signal strength and noise characteristics is present among the one or more input images; and generating a training image and a label image according to the results of the determination based on a preset noise reduction target.

Alternatively, determining whether the image pair is present may include determining a plurality of images having the same signal strength and being noise-independent of each other to be the image pair.

Alternatively, generating the training image and the label image may include: when the image pair is present, determining a first image included in the image pair to be the training image; and generating the label image by combining the first image and a second image according to the noise reduction target.

Alternatively, generating the training image and the label image may include generating the label image such that the first noise of the training image and the second noise of the label image are noise-dependent on each other by as much as the noise reduction target.

Alternatively, generating the training image and the label image may include: when the image pair is not present, determining a first image of the one or more input images to be the label image; and generating the training image based on the first image and the noise reduction target.

Alternatively, generating the training image and the label image may include: causing the signal strength of the training image and the signal strength of the first image to be the same; and generating the training image so that the first noise of the first image and the second noise of the training image are noise-dependent on each other according to the noise reduction target.

Alternatively, the training image and the label image may be input to an artificial neural network model that improves the quality of medical images.

According to an embodiment of the present disclosure for achieving the above-described object, there is disclosed a training data generation method that is performed by a computing device. The training data generation method includes: setting a noise reduction target; and generating a training image based on a first image, and generating a label image by combining the first image and a second image according to the noise reduction target; wherein the first image and the second image have the same signal strength and are noise-independent of each other.

Alternatively, generating the label image may include generating the label image according to the following equation:

$$L = x * Input_1 + (1 - x) * Input_2$$

where:

$L$ = the label image
$x$ = the noise reduction target
$Input_1$ = the first image
$Input_2$ = the second image.

According to an embodiment of the present disclosure for achieving the above-described object, there is disclosed a training data generation method that is performed by a computing device. The training data generation method includes: setting a noise reduction target; and generating a label image based on a first image, and generating a training image based on the first image and the noise reduction target; wherein the training image and the label image have the same signal strength and are noise-dependent on each other by as much as the noise reduction target.

Alternatively, generating the training image may include: generating second noise that has the same signal strength as the first noise of the first image and is noise-independent of the first noise; and generating the training image by combining the first image and the second noise according to the noise reduction target.

Alternatively, generating the training image by combining the first image and the second noise according to the noise reduction target may include generating the training image according to the following equation:

$$T = Input_1 + n_2 \cdot \sqrt{\frac{1}{x} - 1}$$

where:

$T$ = the training image
$Input_1$ = the first image
$n_2$ = the second noise
$x$ = the noise reduction target.

According to an embodiment of the present disclosure for achieving the above-described object, there is disclosed a training data generation device, including: memory configured to store a preset noise reduction target and one or more input images; and a processor configured to: if, among the one or more input image, there is a second image that has the same signal strength as a first image and is noise-independent of the first image, determine the first image to be a training image, and generate a label image by combining the first image and the second image according to the noise reduction target; and, if the second image is not present, determine the first image to be the label image, and generate the training image based on the first image and the noise reduction target.

Alternatively, the training image and the label image have the same signal strength and are noise-dependent on each other by as much as the noise reduction target.

According to an embodiment of the present disclosure for achieving the above-described object, there is disclosed a computer program stored in a computer-readable storage medium. The computer program performs the operations of generating training data when executed on at least one processor. In this case, the operations include operations of: analyzing the signal strength and noise characteristics of one or more input images; determining whether an image pair having corresponding signal strength and noise characteristics is present among the one or more input images; and generating a training image and a label image to be input to an artificial neural network model based on the results of the determination and a noise reduction target set in the artificial neural network model.

**Advantageous Effects**

According to the solution of the present disclosure described above, the present disclosure may finely adjust the degree of noise removal according to a user's demand, thereby preventing image information from being lost due to excessive noise removal.

Furthermore, according to the solution of the present disclosure described above, the present disclosure discloses various methods of generating training and label images according to input image characteristics, so that the time and cost required for preparing training data can be reduced.

**Description of Drawings**

FIG. 1 is a block diagram of a computing device according to an embodiment of the present disclosure;
FIG. 2 is a block diagram of a training data generation device according to an embodiment of the present disclosure;
FIG. 3 is a flowchart of a training data generation method according to an embodiment of the present disclosure;
FIG. 4 is a flowchart showing detailed operations of step S130 of FIG. 3 according to an embodiment of the present disclosure;
FIG. 5 is a flowchart showing detailed operations of step S130 of FIG. 3 according to an embodiment of the present disclosure; and
FIG. 6 is a block diagram of a training data generation device according to an embodiment of the present disclosure.

**Mode for Invention**

Embodiments of the present disclosure will be described in detail below with reference to the accompanying drawings so that those having ordinary skill in the art of the present disclosure (hereinafter referred to as those skilled in the art) can easily implement the present disclosure. The embodiments presented in the present disclosure are provided to enable those skilled in the art to use or practice the content of the present disclosure. Accordingly, various modifications to embodiments of the present disclosure will be apparent to those skilled in the art. That is, the present disclosure may be implemented in various different forms and is not limited to the following embodiments.

The same or similar reference numerals denote the same or similar components throughout the specification of the present disclosure. Additionally, in order to clearly describe the present disclosure, reference numerals for parts that are not related to the description of the present disclosure may be omitted in the drawings.

The term "or" used herein is intended not to mean an exclusive "or" but to mean an inclusive "or." That is, unless otherwise specified herein or the meaning is not clear from the context, the clause "X uses A or B" should be understood to mean one of the natural inclusive substitutions. For example, unless otherwise specified herein or the meaning is not clear from the context, the clause "X uses A or B" may be interpreted as any one of a case where X uses A, a case where X uses B, and a case where X uses both A and B.

The term "and/or" used herein should be understood to refer to and include all possible combinations of one or more of listed related concepts.

The terms "include" and/or "including" used herein should be understood to mean that specific features and/or components are present. However, the terms "include" and/or "including" should be understood as not excluding the presence or addition of one or more other features, one or more other components, and/or combinations thereof.

Unless otherwise specified herein or unless the context clearly indicates a singular form, the singular form should generally be construed to include "one or more."

The term "N-th (N is a natural number)" used herein can be understood as an expression used to distinguish the components of the present disclosure according to a predetermined criterion such as a functional perspective, a structural perspective, or the convenience of description. For example, in the present disclosure, components performing different functional roles may be distinguished as a first component or a second component. However, components that are substantially the same within the technical spirit of the present disclosure but should be distinguished for the convenience of description may also be distinguished as a first component or a second component.

Meanwhile, the term "module" or "unit" used herein may be understood as a term referring to an independent functional unit processing computing resources, such as a computer-related entity, firmware, software or part thereof, hardware or part thereof, or a combination of software and hardware. In this case, the "module" or "unit" may be a unit composed of a single component, or may be a unit expressed as a combination or set of multiple components. For example, in the narrow sense, the term "module" or "unit" may refer to a hardware component or set of components of a computing device, an application program performing a specific function of software, a procedure implemented through the execution of software, a set of instructions for the execution of a program, or the like. Additionally, in the broad sense, the term "module" or "unit" may refer to a computing device itself constituting part of a system, an application running on the computing device, or the like. However, the above-described concepts are only examples, and the concept of "module" or "unit" may be defined in various manners within a range understandable to those skilled in the art based on the content of the present disclosure.

The term "model" used herein may be understood as a system implemented using mathematical concepts and language to solve a specific problem, a set of software units intended to solve a specific problem, or an abstract model for a process intended to solve a specific problem. For example, a neural network "model" may refer to an overall system implemented as a neural network that is provided with problem-solving capabilities through training. In this case, the neural network may be provided with problem-solving capabilities by optimizing parameters connecting nodes or neurons through training. The neural network "model" may include a single neural network, or a neural network set in which multiple neural networks are combined together.

In the present specification, the 'image' may refer to multi-dimensional data composed of discrete image elements (e.g., pixels in a 2D image or voxels in a 3D image). For example, an image may include medical images acquired by medical imaging machines such as a magnetic resonance imaging (MRI) machine, a computed tomography (CT) scanner, an ultrasonic scanner, and an X-ray machine.

The term "medical image archiving and communication system (PACS)" used herein refers to a system that stores, processes, and transmits medical images in accordance with the Digital Imaging and Communications in Medicine (DICOM) standard. For example, the "PACS" operates in conjunction with digital medical imaging equipment, and stores medical images such as magnetic resonance imaging (MRI) images and computed tomography (CT) images in accordance with the DICOM standard. The "PACS" may transmit medical images to terminals inside and outside a hospital over a communication network. In this case, meta information such as reading results and medical records may be added to the medical images.

In the present specification, the 'object' is a target for imaging, and may include a human, an animal, or a part thereof. For example, an object may include a part (organ) of the body or a phantom. The phantom refers to a volume material having a density and an effective atomic number considerably close to those of an organism, and may include a spherical phantom having properties similar to those of the body.

A magnetic resonance image (MRI) system is a system that acquires images of tomographic areas of an object by representing the intensity of a magnetic resonance (MR) signal for a radio frequency (RF) signal generated from a magnetic field having a specific intensity in the form of contrasts between light and darkness.

The MRI system allows a main magnet to form a static magnetic field, and aligns the magnetic dipole moment direction of a specific atomic nucleus of an object, located in the static magnetic field, in the direction of the static magnetic field. A gradient magnetic field coil may generate a gradient magnetic field by applying a gradient signal to the static magnetic field, thereby inducing a different resonance frequency for each portion of the object. An RF coil may radiate a magnetic resonance signal in accordance with the resonance frequency of a portion where an image is to be acquired. Furthermore, as the gradient magnetic field is formed, the RF coil may receive magnetic resonance signals of different resonance frequencies radiated from various portions of the object. The MRI system acquires an image by applying an image reconstruction technique to the magnetic resonance signals received through this step. In addition, the MRI system may reconstruct a plurality of magnetic resonance signals into image data by performing serial or parallel signal processing on the plurality of magnetic resonance signals received by multi-channel RF coils.

The foregoing descriptions of the terms are intended to help to understand the present disclosure. Accordingly, it should be noted that unless the above-described terms are explicitly described as limiting the content of the present disclosure, they should not be interpreted as limiting the technical spirit of the present disclosure.

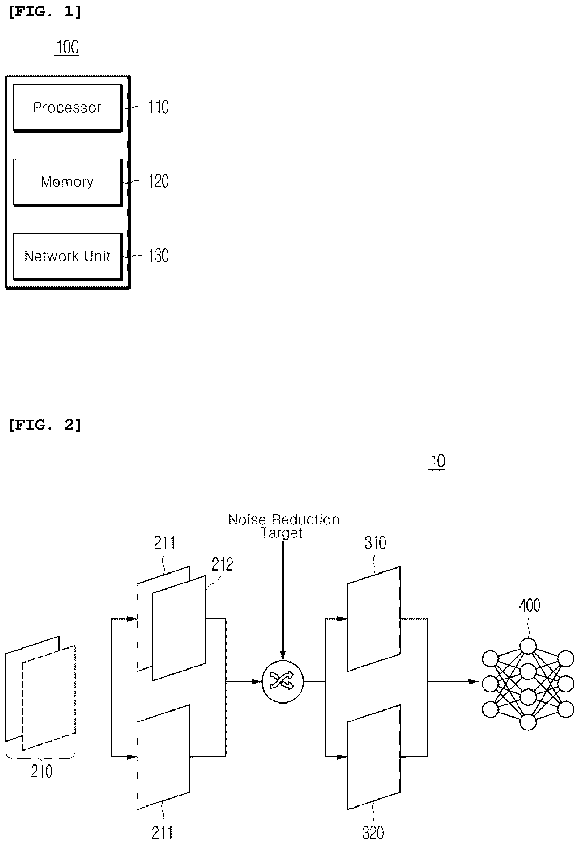

FIG. 1 is a block diagram of a computing device according to an embodiment of the present disclosure.

A computing device 100 according to an embodiment of the present disclosure may be a hardware device or part of a hardware device that performs the comprehensive processing and calculation of data, or may be a software-based computing environment that is connected to a communication network. For example, the computing device 100 may be a server that performs an intensive data processing function and shares resources, or may be a client that shares resources through interaction with a server. Furthermore, the computing device 100 may be a cloud system in which a plurality of servers and clients interact with each other and comprehensively process data. Since the above descriptions are only examples related to the type of computing device 100, the type of computing device 100 may be configured in various manners within a range understandable to those skilled in the art based on the content of the present disclosure.

Referring to FIG. 1, the computing device 100 according to an embodiment of the present disclosure may include a processor 110, memory 120, and a network unit 130. However, FIG. 1 shows only an example, and the computing device 100 may include other components for implementing a computing environment. Furthermore, only some of the components disclosed above may be included in the computing device 100.

The processor 110 according to an embodiment of the present disclosure may be understood as a configuration unit including hardware and/or software for performing computing operation. For example, the processor 110 may read a computer program and perform data processing for machine learning. The processor 110 may process computational processes such as the processing of input data for machine learning, the extraction of features for machine learning, and the calculation of errors based on backpropagation. The processor 110 for performing such data processing may include a central processing unit (CPU), a general purpose graphics processing unit (GPGPU), a tensor processing unit (TPU), an application specific integrated circuit (ASIC), or a field programmable gate array (FPGA). Since the types of processor 110 described above are only examples, the type of processor 110 may be configured in various manners within a range understandable to those skilled in the art based on the content of the present disclosure.

According to an embodiment of the present disclosure, a processor 110 may generate training data that is input to an artificial neural network model. The artificial neural network model is trained using training data, and may output a low-quality image as a high-quality image. For example, the artificial neural network model may generate a high-quality image by removing noise from an input low-quality image.

The artificial neural network model may include at least one neural network. The neural network may include network models such as a Deep Neural Network (DNN), a Recurrent Neural Network (RNN), a Bidirectional Recurrent Deep Neural Network (BRDNN), a Multilayer Perceptron (MLP), and a Convolutional Neural Network (CNN), but is not limited thereto.

The image may be a medical image, e.g., a magnetic resonance image. In this case, the magnetic resonance image may be acquired through accelerated imaging. The accelerated imaging may be understood as imaging techniques that shortens imaging time by increasing the acceleration factor compared to regular imaging. The acceleration factor is a term used in a parallel imaging technique, and may be understood as a value obtained by dividing the number of signal lines fully sampled in the k-space domain by the number of signal lines sampled through imaging.

The processor 110 may generate training data in various manners depending on the number of input images. In the present specification, the training data refers to at least one pair of a training image and a corresponding label image.

The input images may be medical images, magnetic resonance images, or k-space data. The training data may include at least one training image and a label image corresponding to the training image. The label image may have higher quality than the training image. The training image and the label image may be noise-independent of each other by a preset value, and may be noise-dependent on each other by a preset value. In this case, the artificial neural network model trained with the training and label images may be trained to remove noise by a preset value.

The processor 110 may directly set a noise reduction target in the artificial neural network model, or may receive a set noise reduction target value. The processor 110 may generate training data based on the noise reduction target.

The processor 110 analyzes image characteristics of one or more input images. More specifically, the number of input images, signal strength, and noise characteristics are analyzed. The processor 110 determines whether an image pair having corresponding signal strength and noise characteristics is present among one or more input images. Although the image pair refers to two images in the present specification, it is not limited thereto.

Meanwhile, when noise characteristics correspond to each other and signal strength is different, the processor 110 may additionally perform a preprocessing process so that the signal strength of input images having corresponding noise characteristics is the same.

The processor 110 generates training and label images based on the results of the determination and a noise reduction target.

As an example, when an image pair is present among one or more input images, the processor 110 generates a training image and a label image by combining a first image and a second image included in the image pair. When the noise reduction target is set to reduce noise by x times, the processor 110 determines weights corresponding to the first and second images based on x, and combines the first and second images based on the weights.

As an embodiment, when there is one input image or when there is no image pair among input images, the processor 110 generates a training image and a label image using one image, i.e., a first image. More specifically, the processor 110 may generate noise that is independent of the noise of the first image and generate training data using the first image, the generated noise, and the noise reduction target.

Meanwhile, the processor 110 may omit the operation of determining the presence of an image pair and perform the above operations.

According to an embodiment of the present disclosure, the processor 110 may finely adjust the degree of noise removal according to the request of a user. This may prevent image information from being lost due to excessive noise removal. Furthermore, various methods for generating training images and label images according to input image characteristics are disclosed. The time and cost required for preparing training data may be reduced.

According to an embodiment of the present disclosure, the processor 110 may train the artificial neural network model to improve image quality. The image may be, for example, a magnetic resonance image, or may be acquired by accelerated imaging. The accelerated imaging may be understood as an imaging technique that shortens imaging time by increasing the acceleration factor compared to regular imaging. The acceleration factor is a term used in a parallel imaging technique, and may be understood as a value obtained by dividing the number of signal lines fully sampled in the K-space domain by the number of signal lines sampled through imaging. For example, an acceleration factor of 2 can be understood as obtaining a number of signal lines equivalent to half of the number of fully sampled signal lines when lines are obtained by sampling a magnetic resonance signal in the phase encoding direction. Accordingly, as the acceleration factor increases, the imaging time of a magnetic resonance image may decrease proportionally. That is, when the acceleration factor is increased during the imaging of a magnetic resonance image, accelerated imaging in which magnetic resonance image imaging time is shortened may be implemented.

When necessary, the processor 110 may generate a user interface that provides an environment for interaction with the user of the computing device 100 or the user of any client. For example, the processor 110 may generate a user interface that receives one or more input images and the noise reduction target of the artificial neural network model.

The processor 110 may generate a user interface that allows functions such as the output, modification, change, and/or addition of data to be implemented based on an external input signal applied from a user. Since the role of the user interface described above is merely an example, the role of the user interface may be defined in various manners within a range understandable to those skilled in the art based on the content of the present disclosure.

The memory 120 according to an embodiment of the present disclosure may be understood as a configuration unit including hardware and/or software for storing and managing data that is processed in the computing device 100. That is, the memory 120 may store any type of data generated or determined by the processor 110 and any type of data received by the network unit 130. For example, the memory 120 may include at least one type of storage medium of a flash memory type, hard disk type, multimedia card micro type, and card type memory, random access memory (RAM), static random access memory (SRAM), read-only memory (ROM), electrically erasable programmable read-only memory (EEPROM), programmable read-only memory (PROM), magnetic memory, a magnetic disk, and an optical disk. Furthermore, the memory 120 may include a database system that controls and manages data in a predetermined system. Since the types of memory 120 described above are only examples, the type of memory 120 may be configured in various manners within a range understandable to those skilled in the art based on the content of the present disclosure.

The memory 120 may structure, organize, and manage data required for the processor 110 to perform operations, a combination of data, and program code executable on the processor 110. For example, the memory 120 may store one or more input images received through the network unit 130, which will be described later. Furthermore, the memory 120 may store training and label images generated from one or more input images by the processor 110. The memory 120 may store a noise reduction target set in the artificial neural network model. Moreover, the memory 120 may store program code that operates the processor 110 to generate training data.

The network unit 130 according to an embodiment of the present disclosure may be understood as a configuration unit that transmits and receives data through any type of known wired/wireless communication system. For example, the network unit 130 may perform data transmission and reception using a wired/wireless communication system such as a local area network (LAN), a wideband code division multiple access (WCDMA) network, a long term evolution (LTE) network, the wireless broadband Internet (WiBro), a 5th generation mobile communication (5G) network, a ultra wide-band wireless communication network, a ZigBee network, a radio frequency (RF) communication network, a wireless LAN, a wireless fidelity network, a near field communication (NFC) network, or a Bluetooth network. Since the above-described communication systems are only examples, the wired/wireless communication system for the data transmission and reception of the network unit 130 may be applied in various manners other than the above-described examples.

The network unit 130 may receive data required for the processor 110 to perform operations through wired or wireless communication with any system or client. Furthermore, the network unit 130 may transmit data generated through the operation of the processor 110 through wired or wireless communication with any system or any client. For example, the network unit 130 may receive medical images through communication with a PACS, a cloud server for performing tasks such as the improvement of the resolution of medical images and the standardization of medical images, or the computing device 100. The network unit 130 may transmit improved images output from an artificial intelligence model and a mathematical model through communication with the above-described system, server, or computing device 100.

When necessary, the network unit 130 may provide the user interface, generated by the processor 110, to any system or client through wired or wireless communication with the system or client. For example, the network unit 130 may provide a user interface for visualizing data, processed by the processor 110, through communication with a PACS or the computing device 100 including a display to the system or device. The network unit 130 may receive an external input signal, applied from a user, through a user interface from the above-described system or device, and transmit it to the processor 110. In this case, the processor 110 may operate to implement functions such as the output, modification, change, or addition of data based on the external input signal transmitted from the network unit 130. Meanwhile, even without communication through the network unit 130, the system or device provided with a user interface may operate on its own to implement functions such as the output, modification, change, or addition of data according to an external input signal applied from the user.

FIG. 2 is a block diagram of a training data generation device according to an embodiment of the present disclosure.

Referring to FIG. 2, the training data generation device 10 receives one or more input images 210, and generates training data including a training image 310 and a label image 320. The training data generation device 10 may receive a noise reduction target set in the artificial neural network model 400. The artificial neural network model 400 is trained to improve a low-quality image into a high-quality image by the noise reduction target using training data.

The training data generation device 10 may determine whether there is an image pair 211 and 212 having corresponding signal strength and noise characteristics among the one or more input images 210. In this case, the times at which the one or more input images 210 are received may be different from each other. Furthermore, the criteria for determining the image pairs 211 and 212 may be based on various references including an image acquisition method and an image imaging environment, in addition to signal strength and noise characteristics.

When there is the image pair 211 and 212, i.e., a first image 211 and a second image 212 whose image characteristics correspond to each other, the training data generation device 10 generates a training image 310 and a label image 320 by combining the first image 211 and the second image 212 based on the noise reduction target.

When the image pair 211 and 212 is not present or when there is only one input image 210, the training data generation device 10 generates the training image 310 and the label image 320 by using the noise reduction target and the first image 211.

The training data generation device 10 may provide training data including the generated training image 310 and label image 320 to the artificial neural network model 400.

Meanwhile, the configuration shown in FIG. 1 is illustrative. The training data generation device 10 and the artificial neural network model 400 may be included in different systems, respectively. In this case, the training data generation device 10 and the artificial neural network model 400 may transmit and receive training data over a network.

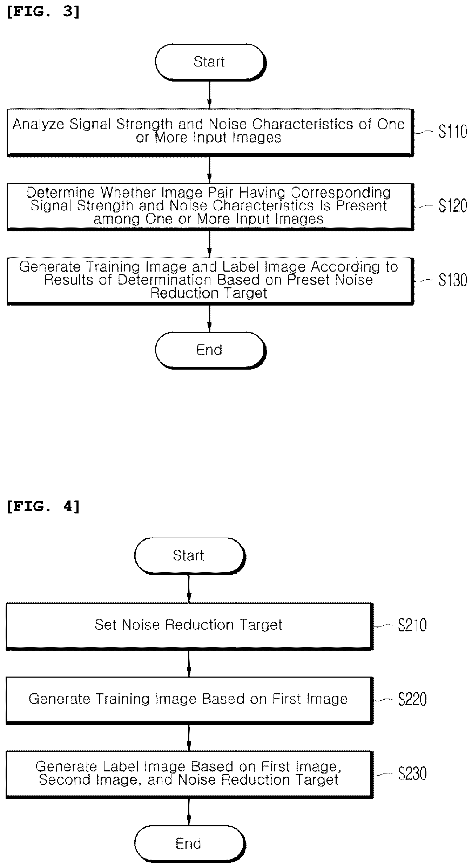

FIG. 3 is a flowchart of a training data generation method according to an embodiment of the present disclosure.

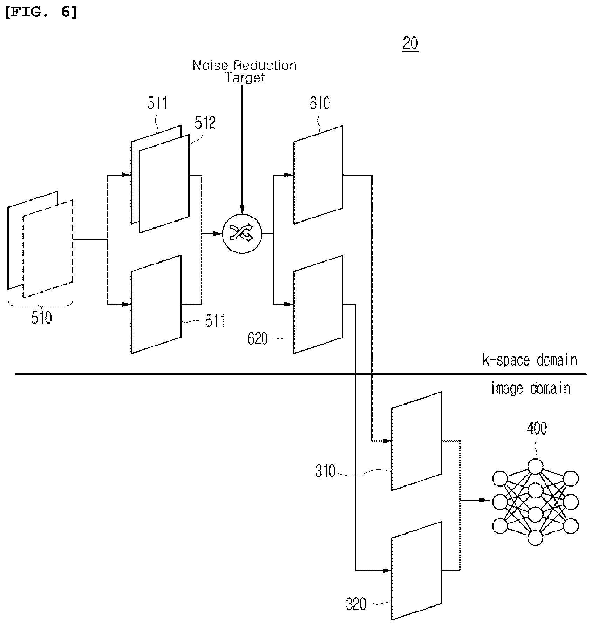

Referring to FIG. 3, the training data generation device may be an embodiment of the training data generation device 10 of FIG. 1 or the training data generation device 20 of FIG. 6.

The training data generation device may set a noise reduction target for the artificial neural network model before step S110. For example, the training data generation device may receive a noise reduction target from a user.

The training data generation device may analyze the signal strength and noise characteristics of one or more input images in step S110. For example, the training data generation device analyzes the signal strength and noise characteristics of the one or more input images. Alternatively, the training data generation device analyzes whether two or more input images are noise-independent of each other.

The training data generation device may determine whether an image pair having corresponding signal strength and noise characteristics is present among the one or more input images in step S120. First and second images included in the image pair have the same signal strength and are noise-independent of each other.

The training data generation device may generate a training image and a label image according to the results of the determination based on the preset noise reduction target in step S130.

More specifically, when an image pair is present, the training data generation device may generate a training image and a label image by combining the first image and the second image included in the image pair. This will be described in detail later with reference to FIG. 4.

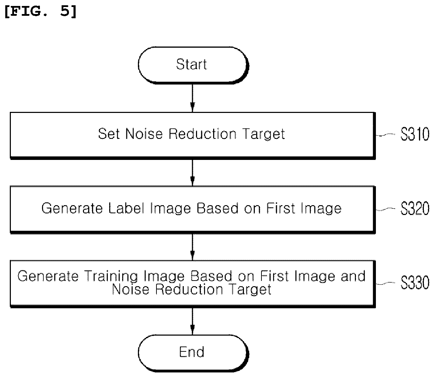

Alternatively, when an image pair is not present, the training data generation device may generate a training image and a label image by using one image, i.e., the first image. This will be described in detail later with reference to FIG. 5.

In this case, the training data generation device may generate a training image and a label image to correspond to the noise reduction target. Furthermore, the training image and the label image are included in the training data input to the artificial neural network model that improves the quality of medical images.

According to an embodiment of the present disclosure, the training data generation device may generate training data in different manners depending on the characteristics of the input images. The time and cost required for preparing training data that satisfies specific conditions may be reduced, and accordingly, the artificial neural network model may be rapidly trained.

FIG. 4 is a flowchart showing detailed operations of step S130 of FIG. 3 according to an embodiment of the present disclosure.

Referring to FIG. 4, the training data generation device may be an embodiment of the training data generation device 10 of FIG. 1 or the training data generation device 20 of FIG. 6. Hereinafter, first and second images may refer to images that are noise-independent of each other and have the same signal strength.

The training data generation device may set a noise reduction target to be applied to the artificial neural network model in step S210. As an example, the noise reduction target may be a real number between 0 and 1.

The training data generation device may generate a training image based on a first image in step S220. As an example, the training data generation device may determine the first image to be a training image.

The training data generation device may generate a label image based on the first image, the second image, and the noise reduction target in step S230. More specifically, the training data generation device may generate a label image by combining the first and second images based on the noise reduction target x. In this case, the noise reduction target x refers to a value that is set to increase the noise by x times or the signal-to-noise ratio (SNR) by $1/x$ times.

That is, the training image and the label image are generated according to Equation 1 below:

$$T = Input_1$$

$$L = x * Input_1 + (1 - x) * Input_2 \qquad (1)$$

In Equation 1, T denotes the training image 310, L denotes the label image 320, $Input_1$ denotes the first image 310, $Input_2$ is the second image 320, and x denotes the noise reduction target.

Meanwhile, the sequential positions of steps S220 and S230 may be changed, and the two steps may be performed in parallel.

Meanwhile, a method of generating a training image and a label image by combining the first image and the second image is not limited to this. That is, there may be various methods of causing the signal strength of a training image and the signal strength of a label image to be the same and setting the noise-independent ratio or noise-dependent ratio between the training image and the label image based on the noise reduction target x.

FIG. 5 is a flowchart showing detailed operations of step S130 of FIG. 3 according to an embodiment of the present disclosure.

Referring to FIG. 5, the training data generation device may be an embodiment of the training data generation device 10 of FIG. 1 or the training data generation device 20 of FIG. 6.

The training data generation device may measure the magnitude of the noise included in a first image, i.e., first noise, before or after step S310. The magnitude of the first noise may refer to the standard deviation of pixel values included in the first image. For example, when the first image is a magnetic resonance image, the background of the first image is segmented, and this means the standard deviation of pixel values of the segmented background. For example, the first image may be an image of the k-space domain, i.e., k-space data. In this case, the magnitude of the first noise refers to the standard deviation of pixel values that are a predetermined distance away from the center of the first image.

The training data generation device may generate a label image based on the first image in step S320. As an example, the training data generation device may determine the first image to be a label image.

The training data generation device may generate a training image based on the first image and the noise reduction target in step S330. More specifically, the training data generation device may generate second noise that has the same magnitude as the first noise and is noise-independent of the first noise. The second noise may be random noise, and the random noise may mean noise following a complex Gaussian distribution, a Rician distribution, or a noncentral chi distribution. More specifically, when the first image is k-space data, second noise following a complex Gaussian distribution is generated. When the first image is a magnetic resonance image, second noise following a Rician distribution or a non-central Chi distribution is generated.

The training data generation device may generate a training image such that the training image and the label image are noise-dependent on each other by as much as the first image, the second noise, and the noise reduction target. That is, the training image and label image are generated according to Equation 2 below:

$$T = Input_1 + \sqrt{\frac{1}{x} - 1} * n_2$$

$$L = Input_1 \qquad (2)$$

In Equation 2, $T$ denotes the training image 310, $L$ denotes the label image 320, $Input_1$ denotes the first image 310, $x$ is the noise reduction target, and $n_2$ denotes the second noise.

Meanwhile, the sequential positions of steps S320 and S330 may be changed, and the two steps may be performed in parallel.

Meanwhile, an operation of pre-processing the first image may be additionally performed. For example, an operation of converting the domain of the first image may be performed.

Thereafter, the training data generation device may provide the training image and the label image to the artificial neural network model. The artificial neural network model may be trained to output high-quality medical images based on low-quality medical images by using the training image and the label image.

FIG. 6 is a block diagram of a training data generation device according to an embodiment of the present disclosure.

Referring to FIG. 6, the training data generation device 20 is similar to the training data generation device 10 of FIG. 2, so that descriptions of common points are omitted. A training image 310 and a label image 320 may be magnetic resonance images in the image domain. One or more input images 510 may be images of the k-space domain, i.e., k-space data.

The training data generation device 20 may generate a k-space training image 610 and a k-space label image 620 that have the same signal strength and are noise-dependent on each other by as much as the noise reduction target.

The training data generation device 20 may determine whether an image pair 511 and 512 is present among the one or more input images 500.

When the first and second images 511 and 512 included in the image pair 511 and 512 are present, the training data generation device 20 generates the k-space training image 610 and a k-space label image 620 by combining the first and second images 511 and 512 based on the noise reduction target. Since the specific details are similar to those of the method described above with reference to FIG. 4, descriptions thereof will be omitted below.

When the image pair 510 is not present or when the input image is only the first image 511, the training data generation device 10 generates the k-space training image 610 and the k-space label image 620 using the first image 511 according to the noise reduction target. Since the specific details are similar to those of the method described above with reference to FIG. 5, the descriptions thereof will be omitted below.

Meanwhile, the training data generation device 20 may generate the k-space training image 610 and the k-space label image 620 using two images or generate the k-space training image 610 and the k-space label image 620 using one image without performing the step of determining whether the image pair 511 and 512 is present.

The training data generation device 20 may generate the training image 310 and the label image 320 by performing Fourier transform on the k-space training image 610 and the k-space label image 620, and may provide the training image 310 and the label image 320 to the artificial neural network model 400.

The foregoing description of the present disclosure is intended for illustrative purposes, and a person having ordinary skill in the art to which the present disclosure pertains will understand that modifications into other specific forms may be easily performed without departing from the technical spirit or essential features of the present disclosure. Therefore, it should be understood that the embodiments described above are all illustrative but not restrictive in all respects. For example, each component described as single may be implemented in a distributed manner, and similarly, components described as distributed may also be implemented in a combined form.

The scope of the present disclosure is defined by the attached claims rather than the detailed description above, and all variations and/or modifications derived from the meanings and scope of the attached claims and their equivalent concepts should be construed as being included in the scope of the present disclosure.

**Claims**

1. A training data generation method, the training data generation method being performed by a computing device including at least one processor, the training data generation method comprising:

   analyzing signal strength and noise characteristics of one or more input images;
   determining whether an image pair having corresponding signal strength and noise characteristics is present among the one or more input images; and
   generating a training image and a label image according to results of the determination based on a preset noise reduction target.

2. The training data generation method of claim 1, wherein determining whether the image pair is present comprises determining a plurality of images having the same signal strength and being noise-independent of each other to be the image pair.

3. The training data generation method of claim 2, wherein generating the training image and the label image comprises:

   when the image pair is present, determining a first image included in the image pair to be the training image; and
   generating the label image by combining the first image and a second image according to the noise reduction target.

4. The training data generation method of claim 3, wherein generating the training image and the label image comprises generating the label image such that first noise of the training image and second noise of the label image are noise-dependent on each other by as much as the noise reduction target.

5. The training data generation method of claim 2, wherein generating the training image and the label image comprises:

   when the image pair is not present, determining a first image of the one or more input images to be the label image; and
   generating the training image based on the first image and the noise reduction target.

6. The training data generation method of claim 5, wherein generating the training image and the label image comprises:

   causing signal strength of the training image and signal strength of the first image to be the same; and
   generating the training image so that first noise of the first image and second noise of the training image are noise-dependent on each other according to the noise reduction target.

7. The training data generation method of claim 2, wherein the training image and the label image are input to an artificial neural network model that improves quality of medical images.

8. A training data generation method, the training data generation method being performed by a computing device including at least one processor, the training data generation method comprising:

   setting a noise reduction target; and
   generating a training image based on a first image, and generating a label image by combining the first image and a second image according to the noise reduction target;
   wherein the first image and the second image have the same signal strength and are noise-independent of each other.

9. The training data generation method of claim 8, wherein generating the label image comprises generating the label image according to the following equation:

$$L = x * Input_1 + (1 - x) * Input_2$$

   where:

   $L$ = the label image
   $x$ = the noise reduction target
   $Input_1$ = the first image
   $Input_2$ = the second image.

10. A training data generation method, the training data generation method being performed by a computing device including at least one processor, the training data generation method comprising:

    setting a noise reduction target; and
    generating a label image based on a first image, and generating a training image based on the first image and the noise reduction target;
    wherein the training image and the label image have the same signal strength and are noise-dependent on each other by as much as the noise reduction target.

11. The training data generation method of claim 10, wherein generating the training image comprises:

    generating second noise that has the same signal strength as first noise of the first image and is noise-independent of the first noise; and
    generating the training image by combining the first image and the second noise according to the noise reduction target.

12. The training data generation method of claim 11, wherein generating the training image by combining the first image and the second noise according to the noise reduction target comprises generating the training image according to the following equation:

$$T = Input_1 + n_2 \cdot \sqrt{\frac{1}{x} - 1}$$

    where:

    $T$ = the training image

$Input_1$ = the first image
$n_2$ = the second noise
$x$ = the noise reduction target.

13. A training data generation device, comprising:

memory configured to store a preset noise reduction target and one or more input images; and
a processor configured to:

if, among the one or more input image, there is a second image that has the same signal strength as a first image and is noise-independent of the first image, determine the first image to be a training image, and generate the label image by combining the first image and the second image according to the noise reduction target; and
if the second image is not present, determine the first image to be the label image, and generate the training image based on the first image and the noise reduction target.

14. The training data generation device of claim 11, wherein the training image and the label image have the same signal strength and are noise-dependent on each other by as much as the noise reduction target.

15. A computer program stored in a computer-readable storage medium, the computer program performing operations of generating training data when executed on at least one processor,
wherein the operations comprise operations of:

analyzing signal strength and noise characteristics of one or more input images;
determining whether an image pair having corresponding signal strength and noise characteristics is present among the one or more input images; and
generating a training image and a label image to be input to an artificial neural network model based on results of the determination and a noise reduction target set in the artificial neural network model.

[FIG. 1]

100

| Processor | ~ 110 |
| Memory | ~ 120 |
| Network Unit | ~ 130 |

[FIG. 2]

10

Noise Reduction
Target

211
212
310
400

210
211
320

[FIG. 3]

```
                    ┌─────────┐
                    │  Start  │
                    └─────────┘
                         │
                         ▼
  ┌────────────────────────────────────────────────┐
  │  Analyze Signal Strength and Noise Characteristics of One  │──── S110
  │            or More Input Images                 │
  └────────────────────────────────────────────────┘
                         │
                         ▼
  ┌────────────────────────────────────────────────┐
  │  Determine Whether Image Pair Having Corresponding  │──── S120
  │  Signal Strength and Noise Characteristics Is Present  │
  │            among One or More Input Images       │
  └────────────────────────────────────────────────┘
                         │
                         ▼
  ┌────────────────────────────────────────────────┐
  │  Generate Training Image and Label Image According to  │──── S130
  │  Results of Determination Based on Preset Noise  │
  │              Reduction Target                   │
  └────────────────────────────────────────────────┘
                         │
                         ▼
                    ┌─────────┐
                    │   End   │
                    └─────────┘
```

[FIG. 4]

```
                    ┌─────────┐
                    │  Start  │
                    └─────────┘
                         │
                         ▼
  ┌────────────────────────────────────────────────┐
  │           Set Noise Reduction Target            │──── S210
  └────────────────────────────────────────────────┘
                         │
                         ▼
  ┌────────────────────────────────────────────────┐
  │      Generate Training Image Based on First Image      │──── S220
  └────────────────────────────────────────────────┘
                         │
                         ▼
  ┌────────────────────────────────────────────────┐
  │   Generate Label Image Based on First Image,    │──── S230
  │   Second Image, and Noise Reduction Target      │
  └────────────────────────────────────────────────┘
                         │
                         ▼
                    ┌─────────┐
                    │   End   │
                    └─────────┘
```

[FIG. 5]

```
                    ┌──────────────┐
                    │    Start     │
                    └──────┬───────┘
                           │
                           ▼
        ┌──────────────────────────────────────┐
        │      Set Noise Reduction Target       │──── S310
        └──────────────────┬───────────────────┘
                           │
                           ▼
        ┌──────────────────────────────────────┐
        │  Generate Label Image Based on First  │──── S320
        │                Image                  │
        └──────────────────┬───────────────────┘
                           │
                           ▼
        ┌──────────────────────────────────────┐
        │ Generate Training Image Based on First│
        │     Image and Noise Reduction Target  │──── S330
        └──────────────────┬───────────────────┘
                           │
                           ▼
                    ┌──────────────┐
                    │     End      │
                    └──────────────┘
```

[FIG. 6]

## INTERNATIONAL SEARCH REPORT

| International application No. |
|---|
| **PCT/KR2023/008603** |

| A. | CLASSIFICATION OF SUBJECT MATTER |
|---|---|

**G16H 30/40**(2018.01)i; **G16H 30/20**(2018.01)i; **G16H 50/70**(2018.01)i; **G16H 50/50**(2018.01)i; **G06V 10/70**(2022.01)i; **A61B 5/055**(2006.01)i

According to International Patent Classification (IPC) or to both national classification and IPC

| B. | FIELDS SEARCHED |
|---|---|

Minimum documentation searched (classification system followed by classification symbols)

G16H 30/40(2018.01); A61B 5/055(2006.01); G06T 5/00(2006.01); G06V 10/70(2022.01); G16H 30/20(2018.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 자기 공명 영상(Magnetic Resonance Imaging), 노이즈(noise), 학습 이미지 (learning image), 라벨 이미지(label image)

| C. | DOCUMENTS CONSIDERED TO BE RELEVANT |
|---|---|

| Category* | Citation of document, with indication, where appropriate, of the relevant passages | Relevant to claim No. |
|---|---|---|
| X<br>A | LEHTINEN, Jaakko et al. Noise2Noise: Learning Image Restoration without Clean Data. arXiv:1803.04189 [cs.CV]. 29 October 2018. [Retrieved on 21 September 2023]. Retrieved from <URL: https://arxiv.org/abs/1803.04189>.<br>See sections 1-3.4 and figure 9. | 1-2,5-7,10-12,15<br>3-4,8-9,13-14 |
| A | KR 10-2428725 B1 (AIRS MEDICAL INC.) 04 August 2022 (2022-08-04)<br>See claims 1 and 5-6 and figures 1-3. | 1-15 |
| A | KR 10-2022-0082302 A (AIRS MEDICAL INC.) 17 June 2022 (2022-06-17)<br>See claims 1-6. | 1-15 |
| A | KR 10-2015-0067835 A (SAMSUNG ELECTRONICS CO., LTD.) 19 June 2015 (2015-06-19)<br>See claims 1-6. | 1-15 |

☑ Further documents are listed in the continuation of Box C. ☑ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **10 October 2023** | **10 October 2023** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**

International application No.

**PCT/KR2023/008603**

### C.    DOCUMENTS CONSIDERED TO BE RELEVANT

| Category* | Citation of document, with indication, where appropriate, of the relevant passages | Relevant to claim No. |
|---|---|---|
| A | KR 10-2019-0038333 A (INDUSTRY-ACADEMIC COOPERATION FOUNDATION, YONSEI UNIVERSITY) 08 April 2019 (2019-04-08)<br>   See claim 1 and figure 2. | 1-15 |
| PX | KR 10-2533000 B1 (AIRS MEDICAL INC.) 16 May 2023 (2023-05-16)<br>   See entire document.<br>   (Note: This document is a published earlier application that serves as a basis for claiming priority of the present international application.) | 1-15 |

Form PCT/ISA/210 (second sheet) (July 2022)

| Patent document cited in search report | | | Publication date (day/month/year) | Patent family member(s) | | | Publication date (day/month/year) |
|---|---|---|---|---|---|---|---|
| KR | 10-2428725 | B1 | 04 August 2022 | None | | | |
| KR | 10-2022-0082302 | A | 17 June 2022 | None | | | |
| KR | 10-2015-0067835 | A | 19 June 2015 | KR | 10-1629162 | B1 | 21 June 2016 |
| | | | | US | 10317496 | B2 | 11 June 2019 |
| | | | | US | 2015-0160319 | A1 | 11 June 2015 |
| KR | 10-2019-0038333 | A | 08 April 2019 | KR | 10-2210457 | B1 | 01 February 2021 |
| | | | | US | 10989779 | B2 | 27 April 2021 |
| | | | | US | 2019-0101605 | A1 | 04 April 2019 |
| KR | 10-2533000 | B1 | 16 May 2023 | None | | | |

Form PCT/ISA/210 (patent family annex) (July 2022)